(19) Europäisches Patentamt
European Patent Office
Office européen des brevets

(11) EP 0 929 285 B1

(12) EUROPÄISCHE PATENTSCHRIFT

(45) Veröffentlichungstag und Bekanntmachung des Hinweises auf die Patenterteilung:
**21.11.2001 Patentblatt 2001/47**

(21) Anmeldenummer: **97909334.1**

(22) Anmeldetag: **29.09.1997**

(51) Int Cl.[7]: **A61K 7/06**, A61K 7/48

(86) Internationale Anmeldenummer:
**PCT/EP97/05354**

(87) Internationale Veröffentlichungsnummer:
**WO 98/14164 (09.04.1998 Gazette 1998/14)**

(54) **VERWENDUNG WASSERLÖSLICHER COPOLYMERE ALS WIRKSTOFFE IN KOSMETISCHEN FORMULIERUNGEN**

USE OF WATER-SOLUBLE COPOLYMERS AS ACTIVE INGREDIENTS IN COSMETICS

UTILISATION DE COPOLYMERES SOLUBLES DANS L'EAU COMME INGREDIENTS ACTIFS DE PRODUITS COSMETIQUES

(84) Benannte Vertragsstaaten:
**AT BE CH DE ES FI FR GB IT LI NL SE**

(30) Priorität: **30.09.1996 DE 19640363**

(43) Veröffentlichungstag der Anmeldung:
**21.07.1999 Patentblatt 1999/29**

(73) Patentinhaber: **BASF AKTIENGESELLSCHAFT**
**67056 Ludwigshafen (DE)**

(72) Erfinder:
- **NIESSNER, Manfred**
  **D-67105 Schifferstadt (DE)**
- **NILZ, Claudia**
  **D-67127 Rödersheim-Gronau (DE)**
- **HÖSSEL, Peter**
  **D-67105 Schifferstadt (DE)**
- **KOTHRADE, Stephan**
  **D-67117 Limburgerhof (DE)**
- **SANNER, Axel**
  **D-67227 Frankenthal (DE)**

(74) Vertreter: **Kinzebach, Werner, Dr. et al**
**Patentanwälte,**
**Reitstötter, Kinzebach & Partner,**
**Ludwigsplatz 4**
**67059 Ludwigshafen (DE)**

(56) Entgegenhaltungen:
**EP-A- 0 510 246**
**WO-A-96/03969**
**DE-A- 1 495 692**
**DE-B- 1 151 940**

- **MARTINO G ET AL: "NOVEL APPROACHES TO HAIR STYLING PRODUCTS" PARFUEMERIE UND KOSMETIK, Bd. 76, Nr. 10, Oktober 1995, Seiten 616-619, XP000671065**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 929 285 B1

## Beschreibung

Die Erfindung betrifft die Verwendung wasserlöslicher Copolymere als Bestandteile kosmetischer Mittel sowie kosmetische Mittel, welche diese Copolymere enthalten.

Polymere finden in Kosmetik und Medizin vielfache Anwendung. In Seifen, Cremes und Lotionen beispielsweise dienen sie in der Regel als Formulierungshilfsmittel, z.B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik dagegen besteht darin, die Eigenschaften des Haares zu beeinflussen. So werden Conditioner dazu eingesetzt, um die Trokken- und Naßkämmbarkeit, Gefühl, Glanz und Erscheinungsform zu verbessern sowie dem Haar antistatische Eigenschaften zu verleihen. Weiterhin können sie verfestigend wirken, indem sie auf dem Haar hydrophobe Filme ausbilden.

Bevorzugt werden wasserlösliche Polymere mit polaren, häufig kationischen Funktionalitäten eingesetzt, die eine größere Affinität zur strukturell bedingten negativen Oberfläche des Haares aufweisen. Struktur und Wirkungsweise verschiedener Haarbehandlungspolymere sind in Cosmetics & Toiletries 103 (1988) 23 beschrieben. Handelsübliche Conditionerpolymere sind z.B. kationische Hydroxyethylcellulose, kationische Polymere auf der Basis von N-Vinylpyrrolidon, Acrylamid und Diallyldimethylammoniumchlorid oder Silikone.

In der US 4,713,236 sind Haarconditioner auf Basis von Vinylamineinheiten enthaltenden Polymeren beschrieben. Genannt sind dabei insbesondere Polyvinylamin sowie dessen Salze, $\alpha$-substituierte Polyvinylamine wie z.B. Poly-($\alpha$-amino-acrylsäure) oder auch Copolymere, die neben Vinylamin Comonomere wie Vinylalkohol, Acrylsäure, Acrylamid, Maleinsäureanhydrid, Vinylsulfonat und 2-Acrylamido-2-methylpropan-sulfonsäure einpolymerisiert enthalten. Die eventuelle Anwendbarkeit von Copolymeren mit wiederkehrenden Vinylamin- und Vinylcarbonsäureamideinheiten als wirksamer Bestandteil eines Haarconditioners wird in diesem Dokument nicht untersucht.

Die WO-A-96/03969 beschreibt Haarpflegemittel, enthaltend ein N-Vinylformamid-Homopolymer oder ein Copolymer aus N-Vinylformamid-Einheiten und einem weiteren Vinylmonomer, ausgewählt unter Styrolen, Alkylestern von Acryl- und Methacrylsäure, Vinylestern der Formel $CH_2$=CH-OCO Alkyl, N-Alkyl-substituierten Acryl- und Methacrylamiden, Estern von Fumar-, Ithacon- und Maleinsäure, Vinylethern, Hydroxy-funktionalisierten Acrylaten und Methacrylaten, Acrylamid, nicht-Alkyl-substituierten Acrylamiden und cyclischen Amiden. Als konkretes Beispiel für ein cyclisches Amid wird Vinylpyrrolidon genannt. Weitere Beispiele für Vinylmonomere sind sekundäre, tertiäre und quaternäre Amine, wie Dimethyl-diallylammoniumchlorid, t-Butylaminoethylmethacrylat, Dimethylaminoethylmethacrylat, Diethylaminoethylmethacrylat, Dimethylaminopropylmethacrylat und die quaternisierten Derivate davon.

Die JP 06 122 725 beschreibt pulverförmige N-Vinylformamidhomo- und -copolymere. Diese Polymere finden Verwendung z.B. als Bestandteil von Tonern, Chromatographiematerialien, Träger für Immunodiagnostica und Füllstoffe für Farben und Kosmetika. Eine Eignung dieser Polymere als Konditioniermittel, insbesondere als Konditioniermittel für Shampoos, wird nicht beschrieben.

Hydrolysierte Oligomere von N-Vinylformamid sind aus der US 5,373,076 bekannt. Als hypothetische Anwendungsgebiete für diese Polymere werden unter anderem genannt Klebstoffe, Bindemittel, Wasserbehandlung, Papierherstellung, Erdöl- und Mineralienförderung, Medizin und Körperpflege.

Aus der EP 510 246 sowie der japanischen Schrift JP 3223 304 sind vernetzte, vorwiegend anionische Homo- und Copolymerisate von N-Vinylcarbonsäureamiden bekannt, die als Wasserabsorber und Verdicker, beispielsweise im Kosmetikbereich, eingesetzt werden.

Copolymerisate aus Acrylamid und (Meth)Acrylsäure, die als weitere Monomere auch N-Vinylcarbonsäureamide enthalten können, finden gemäß DE 34 27 220 ebenfalls Anwendung als Verdicker in kosmetischen oder pharmazeutischen Zubereitungen.

Die EP 452 758 beschreibt kohlenwasserstoffreiche Gele für dermale kosmetische und medizinische Anwendungen, die neben verschiedenen Tensiden als weiteren Bestandteil ein wasserlösliches Polymer, wie z.B. Polyvinylformamid, enthalten.

Körperpflegecremes, die ein Monoaldehyd-modifiziertes Vinylaminpolymer enthalten, sind aus der US 5,270,379 bekannt.

Copolymere, die beispielsweise als Haarfestiger Verwendung finden und aufgebaut sind aus N-Vinylamidmonomeren der Formel

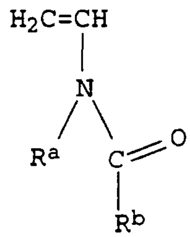

worin $R^a$ und $R^b$ für H oder $C_1$-$C_5$-Alkyl stehen, und das Comonomer ausgewählt ist unter Vinylethern, Vinyllactamen, Vinylhalogeniden, Vinylestern einbasiger, gesättigter Carbonsäuren, (Meth)acrylsäureestern, -amiden und -nitrilen, und Estern, Anhydriden und Imiden der Maleinsäure, sind aus der DE 14 95 692 bekannt.

Die GB 1,082,016 beschreibt Homopolymere von N-Vinyl-N-methylacetamid bzw. Copolymere dieser Verbindung mit Vinylestern, Vinylethern, (Meth)acrylsäureestern, -amiden und -nitrilen, von Malein- und Fumarsäure abgeleiteten Vinylverbindungen, Styrol, Butadien und Vinylhalogeniden, und deren Verwendung in Shampoo-, Festiger- und Haarsprayformulierungen.

Der vorliegenden Erfindung liegt die Aufgabe zugrunde, neue Polymerisate mit verbesserter Wirksamkeit in kosmetischen Zubereitungen zur Verfügung zu stellen.

Die Aufgabe wird erfindungsgemäß gelöst durch Verwendung von wasserlöslichen Copolymeren, die als charakteristische Strukturelemente

a) Vinylcarbonsäureamid-Einheiten der allgemeinen Formel I

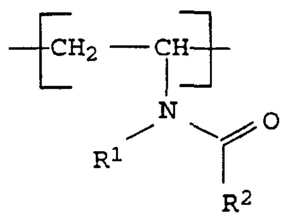

(I)

worin
$R^1$ und $R^2$ unabhängig voneinander für H, Alkyl, Cycloalkyl, Aryl oder Aralkyl stehen, und

b) heterocyclische Einheiten, ausgewählt unter

i)

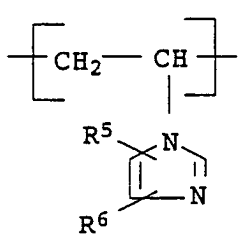

(III)

worin
$R^5$ und $R^6$ unabhängig voneinander ausgewählt sind unter H, Alkyl, Aryl, Aralkyl oder zusammen einen benzannelierten Ring bedeuten; und

ii)

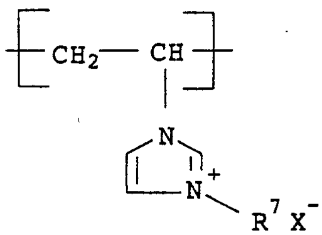

(IV)

worin
X für den Rest einer anorganischen oder organischen Säure steht, und $R^7$ für H, Alkyl oder Aralkyl steht; als Bestandteil kosmetischer Mittel.

Vorzugsweise besitzen die erfindungsgemäß verwendeten Copolymere ein gewichtsmittleres Molekulargewicht im Bereich von etwa $10^3$ bis $10^8$ g/mol, vorzugsweise etwa $10^4$ bis $10^7$ g/mol.

Soweit keine anderen Angaben gemacht werden, gelten bei der konkreten Beschreibung der Erfindung die folgenden Definitionen:

Erfindungsgemäß verwendbare Alkylreste umfassen geradkettige oder verzweigte, gesättigte Kohlenstoffketten mit 1 bis 12 Kohlenstoffatomen. Beispielsweise können folgende Reste genannt werden: $C_1$-$C_6$-Alkylreste, wie Methyl, Ethyl, n-Propyl, i-Propyl, n-Butyl, sec.Butyl, 2- oder 3-Methyl-pentyl; und längerkettige Reste, wie unverzweigtes Heptyl, Octyl, Nonyl, Decyl, Undecyl, Lauryl und die ein- oder mehrfach verzweigten Analoga davon.

Erfindungsgemäß brauchbare Alkylenreste umfassen geradkettige $C_1$-$C_{10}$-Alkylenreste, wie z.B. Methylen, Ethylen, Propylen, Butylen, Pentylen und Hexylen sowie verzweigte $C_1$-$C_{10}$-Alkylenreste, wie z.B. 1,1-Dimethylbutylen, 1,3-Dimethylbutylen, 1,1-Dimethylbutylen, 1,2-Dimethylpentylen und 1,3-Dimethylhexylen. Bevorzugt sind jedoch geradkettige Alkylengruppen mit 1 bis 6 Kohlenstoffatomen.

Erfindungsgemäß verwendbare Cycloalkylreste umfassen insbesondere $C_3$-$C_{12}$-Cycloalkylreste, wie z.B. Cyclopropyl, Cyclobutyl, Cyclopentyl, Cyclohexyl, Cycloheptyl, Cyclooctyl, Cyclopropylmethyl, Cyclopropylethyl, Cyclopropylpropyl, Cyclobutylmethyl, Cyclobutylethyl, Cyclopentylethyl, -propyl, -butyl, -pentyl, -hexyl und dergleichen.

Erfindungsgemäß geeignete Aralkylreste umfassen Phenyl- und Naphthyl-$C_1$-$C_{12}$-alkylreste, wobei der $C_1$-$C_{12}$-Alkylteil wie oben angegeben definiert ist. Bevorzugte Aralkylreste sind Phenyl-$C_1$-$C_6$-alkylreste.

Erfindungsgemäß geeignete Arylreste sind beispielsweise Phenyl und Naphthyl.

Bevorzugte Gegenionen $X^-$ sind $Cl^-$, $Br^-$, $H_2PO_4^-$, $SO_4^{2-}$, $NO_3^-$, $HCOO^-$, Phenyl-$SO_3^-$.

Bevorzugte Beispiele für Monomere der Formel I sind N-Vinylformamid, N-Vinyl-N-methylformamid, N-Vinyl-N-ethylformamid, N-Vinyl-N-propylformamid, N-Vinyl-N-isopropylformamid, N-Vinyl-N-butylformamid, N-Vinyl-N-sek.-butylformamid, N-Vinyl-N-tert.-butylformamid, N-Vinyl-N-pentylformamid, N-Vinylacetamid, N-Vinyl-N-methylacetamid und N-Vinyl-N-ethylacetamid.

Die heterocyclischen Monomere sind ausgewählt unter Imidazol- und Imidazolium- Monomeren der folgenden Formeln III und IV:

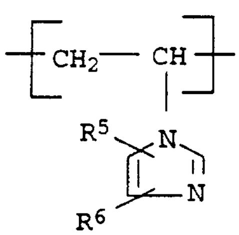

(III)

worin
$R^5$ und $R^6$ unabhängig voneinander ausgewählt sind unter H, Alkyl, Aryl, Aralkyl oder zusammen einen benzannellierten Ring bedeuten;

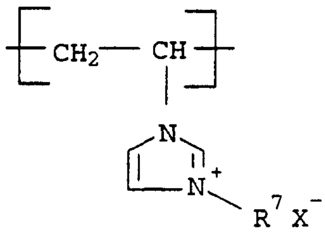

(IV)

worin

X für den Rest einer anorganischen oder organischen Säure steht und $R^7$ für H, Alkyl oder Aralkyl steht.

Besonders bevorzugte Monomere der Formel III sind Verbindungen, worin $R^5$ und $R^6$ unabhängig voneinander für H oder $C_1$-$C_6$-Alkyl, insbesondere für H stehen. Besonders bevorzugte Monomere der Formel IV sind Verbindungen, worin $R^7$ für $C_1$-$C_6$-Alkyl oder X für Cl oder Br steht.

Beispiele für Imidazol- und Imidazolin-Monomere sind N-Vinyl-2-methylimidazol, N-Vinyl-4-methylimidazol, N-Vinyl-5-methylimidazol, N-Vinyl-2-ethylimidazol, N-Vinyl-N'methyl-imidazoliumchlorid, N-Vinyl-N'-ethyl-imidazoliumchlorid, N-Vinylimidazolin, N-Vinyl-2-methylimidazolin sowie N-Vinyl-2-ethylimidazolin.

Bevorzugt sind außerdem Copolymere, enthaltend

a) 0,1 bis 99,9, vorzugsweise 1 bis 99, insbesondere 5 bis 95 Mol-% N-Vinylcarbonsäureamid-Einheiten der Formel I,

b) 0,1 bis 99,9, vorzugsweise 1 bis 99, insbesondere 5 bis 95 Mol-% Einheiten der Formel III und/oder IV,

c) 0 bis 99,8, vorzugsweise 0 bis 98, insbesondere 0 bis 90 Mol-% von a) und b) verschiedener monoethylenisch ungesättigter Monomereinheiten; und

d) 0 bis 5, vorzugsweise 0 bis 4, insbesondere 0 bis 3 Mol-% Monomereinheiten mit mindestens zwei ethylenisch ungesättigten Doppelbindungen.

Beispiele für Monomere c) sind Vinylamine und deren Salze, wie z.B. Vinylammoniumhalogenide, Vinylalkohol, Vinylformiat, Vinylacetat, Vinylpropionat, Vinylbutyrat, N-Vinylpyrrolidon, N-Vinylcaprolactam, N-Vinylharnstoffe, $C_1$-$C_6$-Vinylether, monoethylenisch ungesättigte $C_3$-$C_8$- Carbonsäuren und -Dicarbonsäuren sowie deren Ester, Amide, Anhydride und Nitrile, Olefine sowie Vinylaromaten und sulfonsäuregruppen enthaltende Monomere mit polymerisier barer Doppelbindung.

Geeignete zusätzliche ethylenisch ungesättigte Monomere c) sind Vinylformiat, Vinylacetat, Vinylpropionat, Vinylbutyrat, ungesättigte $C_3$-bis $C_8$-Carbonsäuren, wie z.B. Acrylsäure, Methacrylsäure, Maleinsäure, Crotonsäure, Itaconsäure und Vinylessigsäure sowie deren Alkali- und Erdalkalimetallsalze, Ester, Amide und Nitrile, beispielsweise Methylacrylat, Methylmethacrylat, Ethylacrylat, Ethylmethacrylat, Propylacrylat und Butylacrylat oder Glykol- bzw. Polyglykolester ethylenisch ungesättigter Carbonsäuren, wobei jeweils nur eine OH-Gruppe der Glykole und Polyglykole verestert ist, z.B. Hydroxyethylacrylat, Hydroxyethylmethacrylat, Hydroxypropylacrylat, Hydroxypropylmethacrylat, Hydroxybutylacrylat, Hydroxybutylmethacrylat oder auch Acrylsäure- und Methacrylsäuremonoester von Polyalkylenglykolen eines Molgewichts von 1500 bis 10000. Geeignet sind auch Ester von ethylenisch ungesättigten Carbonsäuren mit Aminoalkoholen, wie z. B. Dimethylaminoethylacrylat, Dimethylaminoethylmethacrylat, Diethylaminoethylacrylat, Diethylaminoethylmethacrylat, Dimethylaminopropylacrylat, Dimethylaminopropylmethacrylat, Diethylaminopropylacrylat, Diethylaminopropylmethacrylat, Dimethylaminobutylacrylat und Diethylaminobutylacrylat. Die basischen Acrylate liegen dabei in Form der freien Basen, der Salze mit Mineralsäuren, wie z.B. Salzsäure, Schwefelsäure und Salpetersäure, der Salze organischer Säuren, wie Ameisensäure oder Benzolsulfonsäure, oder in quaternisierter Form vor.

Geeignete Quaternisierungsmittel sind beispielsweise Dimethylsulfat, Diethylsulfat, Methylchlorid, Ethylchlorid oder Benzylchlorid.

Außerdem eignen sich als Monomere c) ungesättigte Amide wie beispielsweise Acrylamid, Methacrylamid sowie N-Alkylmono- und -diamide mit Alkylresten von 1 bis 6 C-Atomen, z.B. N-Methyl-acrylamid, N,N-Dimethylacrylamid, N-Methylmethacrylamid, N-Ethylacrylamid, N-Propylacrylamid und tert. Butylacrylamid sowie basische (Meth-) acrylamide, wie z. B. Dimethylaminoethylacrylamid, Dimethylaminoethylmethacrylamid, Diethylaminoethylacrylamid, Diethylaminoethylmethacrylamid, Dimethylaminopropylacrylamid, Dimethylaminopropylmethacrylamid, Diethylaminopropylacrylamid und Diethylaminopropylmethacrylamid.

Auch $C_1$-$C_6$-Vinylether, wie z.B. Vinylmethylether, Vinylethylether, Vinylpropylether, Vinylisopropylether, Vinylbutylether, Vinylisobutylether, Vinylpentylether und Vinylhexylether können als Monomere c) eingesetzt werden.

Weiterhin kommen als Monomere c) in Frage N-Vinylpyrrolidon, N-Vinylcaprolactam, N-Vinylharnstoff und

substituierte N-Vinylharnstoffe, beispielsweise N-Vinyl-N'-methylharnstoff und N-Vinyl-N'-dimethylharnstoff.

Als Monomere c) können auch monoethylenisch ungesättigte Verbindungen mit Sulfogruppen eingesetzt werden, wie beispielsweise Vinylsulfonsäure, Allylsulfonsäure, Methallylsulfonsäure, Styrolsulfonsäure oder Acrylsäure-3-sulfopropylester, Methacrylsäure-3-sulfopropylester und 2-Acrylamido-2-methylpropansulfonsäure. Die Säuregruppen aufweisenden Verbindungen können in Form der freien Säuren, der Ammonium-, Alkalimetall- und Erdalkalimetallsalze vorliegen.

Eine weitere Modifizierung der Copolymerisate kann dadurch erzielt werden, daß man 0 bis 5 Mol-%-Einheiten von Monomeren d) mit mindestens zwei ethylenisch ungesättigten nicht konjugierten Doppelbindungen mit einpolymerisiert. Derartige Comonomere werden üblicherweise bei Copolymerisationen als Vernetzer verwendet. Die Mitverwendung dieser Comonomere während der Copolymerisation bewirkt eine Erhöhung der Molmassen der Copolymerisate. Geeignete Verbindungen dieser Art sind beispielsweise Methylenbisacrylamid, Ester von Acrylsäure und Methacrylsäure mit mehrwertigen Alkoholen, z.B. Glykoldiacrylat, Glycerintriacrylat, Glykoldimethacrylat, Glycerintrimethacrylat sowie mindestens zweifach mit Acrylsäure oder Methacrylsäure veresterte Polyole, wie Pentaerythrit und Glucose. Geeignete Vernetzer sind außerdem Divinylbenzol, Divinyldioxan, Pentaerythrittriallylether, Pentaallylsucrose, Divinylharnstoff und Divinylethylenharnstoff.

Die erfindungsgemäß einzusetzenden Copolymerisate erhält man nach literaturbekanntem Verfahren durch radikalisch initiierte Copolymerisation von Monomeren der Formeln I (Monomere a)) und der Formel II (Monomere b)) und gegebenenfalls in Anwesenheit der obengenannten weiteren Monomere c) und/oder d) und eventuell anschließender partieller Abspaltung der Carbonyl-Gruppierung durch Hydrolyse. Geeignete Herstellungsverfahren sind beispielsweise beschrieben in den EP-A-0 071 050, EP-A-0 251 182 und der EP-A-0 216 387, worauf hiermit ausdrücklich Bezug genommen wird.

Die Polymerisation kann in Gegenwart oder auch in Abwesenheit eines inerten Lösungs- oder Verdünnungsmittels durchgeführt werden. Da die Polymerisation in Abwesenheit von inerten Lösungs- oder Verdünnungsmitteln meistens zu uneinheitlichen Polymerisaten führt, ist die Polymerisation in einem inerten Lösungs- oder Verdünnungsmittel bevorzugt. Geeignet sind beispielsweise solche inerten Lösungsmittel, in denen die offenkettigen N-Vinylcarbonsäureamide löslich sind. Für die Lösungspolymerisation eigenen sich z.B. inerte Lösungsmittel, wie Methanol, Ethanol, Isopropanol, n-Propanol, n-Butanol, Tetrahydrofuran, Dioxan, Wasser sowie Mischungen der genannten inerten Lösungsmittel. Die Polymerisation kann kontinuierlich oder diskontinuierlich durchgeführt werden.
Sie erfolgt in Gegenwart von radikalbildenden Initiatoren, die in Mengen von 0,01 bis 20, vorzugsweise 0,05 bis 10 Gew.-%, bezogen auf die Monomeren, eingesetzt werden. Gegebenenfalls kann die Polymerisation auch allein durch die Einwirkung von energiereicher Strahlung, z.B. Elektronenstrahlen oder UV-Strahlen initiiert werden.

Um Polymerisate mit niedrigen Molekulargewichten z.B. von 1000 bis 100000, vorzugsweise 5000 bis 50000 herzustellen, wird die Polymerisation zweckmäßigerweise in Gegenwart von Reglern durchgeführt. Geeignete Regler sind beispielsweise organische Verbindungen, die Schwefel in gebundener Form enthalten. Hierzu gehören Mercaptoverbindungen, wie Mercaptoethanol, Mercaptopropanol, Mercaptobutanol, Mercaptoessigsäure, Mercaptopropionsäure, Butylmercaptan, Dodecylmercaptan. Als Regler eignen sich außerdem Allylverbindungen, wie Allylalkohol, Aldehyde, wie Formaldehyd, Acetaldehyd, Propionaldehyd, n-Butyraldehyd und Isobutyraldehyd, Ameisensäure, Ammoniumformiat, Propionsäure, Hydrazinsulfat und Butenole.

Falls die Polymerisation in Gegenwart von Reglern durchgeführt wird, benötigt man davon 0,05 bis 20 Gew.-%, bezogen auf die bei der Polymerisation eingesetzten Monomeren.

Die Polymerisation der Monomeren erfolgt üblicherweise in einer Inertgasatmosphäre unter Ausschluß von Luftsauerstoff. Während der Polymerisation wird im allgemeinen für eine gute Durchmischung der Reaktionsteilnehmer gesorgt. Bei kleineren Ansätzen, bei denen eine sichere Abführung der Polymerisationswärme gewährleistet ist, kann man die Monomeren diskontinuierlich polymerisieren, indem man das Reaktionsgemisch auf die Polymerisationstemperatur erhitzt und dann die Reaktion ablaufen läßt. Diese Temperaturen liegen dabei im Bereich von 40 bis 180°C, wobei unter Normaldruck, vermindertem oder auch erhöhtem Druck gearbeitet werden kann. Polymerisate mit einem hohen Molekulargewicht erhält man, wenn man die Polymerisation in Wasser durchführt. Dies kann beispielsweise zur Herstellung wasserlöslicher Polymerisate in wäßriger Lösung, als Wasser-in-Öl-Emulsion oder nach dem Verfahren der umgekehrten Suspensionspolymerisation erfolgen.

Um eine Verseifung der monomeren N-Vinylcarbonsäureamide während der Polymerisation in wäßriger Lösung zu vermeiden, führt man die Polymerisation vorzugsweise in einem pH-Bereich von 4 bis 9, insbesondere von 5 bis 8 durch. In vielen Fällen empfiehlt es sich, zusätzlich noch in Gegenwart von Puffern zu arbeiten, z.B. primärem oder sekundärem Natriumphosphat.

Aus den oben beschriebenen Polymerisaten erhält man gegebenenfalls durch partielle Abspaltung der Gruppierung

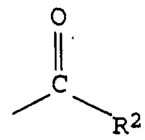

aus den N-Vinylcarbonsäureamideinheiten unter Bildung von Amin- bzw. Ammoniumgruppen erfindungsgemäß brauchbare Copolymerisate, die als Monomer c) ein Vinylamin oder dessen entsprechende Ammoniumverbindung enthalten. Die Hydrolyse wird vorzugsweise in Wasser unter Einwirkung von Säuren, Basen oder Enzymen durchgeführt, kann jedoch auch in Abwesenheit der genannten Hydrolysemittel erfolgen. In Abhängigkeit von den Reaktionsbedingungen bei der Hydrolyse, d.h. der Menge an Säure oder Base, bezogen auf das zu hydrolysierende Polymerisat, sowie Reaktionszeit und -temperatur, erhält man verschiedene Hydrolysegrade. Die Hydrolyse wird soweit geführt, daß 0,1 bis 99,9 Mol-%, bevorzugt 1 bis 99 Mol-% und ganz besonders bevorzugt 5 bis 95 Mol-% der Carbonsäureamidreste hydrolytisch gespalten werden.

Für die Hydrolyse geeignete Säuren sind beispielsweise Mineralsäuren, wie Halogenwasserstoff (gasförmig oder in wäßriger Lösung), Schwefelsäure, Salpetersäure, Phosphorsäure (ortho-, meta- oder Polyphosphorsäure) oder organische Säuren, z.B. $C_1$- bis $C_5$-Carbonsäuren wie Ameisensäure, Essigsäure oder Propionsäure oder aliphatische und aromatische Sulfonsäuren, wie Methansulfonsäure, Benzolsulfonsäure oder Toluolsulfonsäure. Bei der Hydrolyse mit Säuren beträgt der pH-Wert 0 bis 5. Pro abzuspaltenden Carbonsäurerest im Polymerisat benötigt man 0,05 bis 1,5 Äquivalente an Säure, vorzugsweise 0,4 bis 1,2.

Bei der Hydrolyse mit Basen können Metallhydroxide von Metallen der ersten und zweiten Hauptgruppe des Periodischen Systems verwendet werden, beispielsweise Lithiumhydroxid, Natriumhydroxid, Kaliumhydroxid, Calciumhydroxid, Strontiumhydroxid und Bariumhydroxid. Ebenso eigenen sich aber auch Ammoniak oder Alkylderivate des Ammoniaks, z.B. Alkyl- oder Arylamine, wie Triethylamin, Monoethanolamin, Diethanolamin, Triethanolamin, Morpholin, Piperiden, Pyrrolidin oder Anilin. Bei der Hydrolyse mit Basen beträgt der pH-Wert 8 bis 14. Die Basen können in festem, flüssigem oder gegebenenfalls auch gasförmigem Zustand, verdünnt oder unverdünnt eingesetzt werden. Vorzugsweise verwendet man Ammoniak, Natronlauge oder Kalilauge. Die Hydrolyse im sauren oder alkalischen pH-Bereich erfolgt bei Temperaturen von 20 bis 170°C, vorzugsweise 50 bis 120°C. Sie ist nach etwa 2 bis 8, vorzugsweise 3 bis 5 Stunden beendet.

Besonders bewährt hat sich eine Verfahrensweise, bei der die Säuren oder Basen in wäßriger Lösung zugesetzt werden. Nach der Hydrolyse führt man i.a. eine Neutralisation durch, so daß der pH-Wert der hydrolysierten Polymerlösung 2 bis 8, vorzugsweise 3 bis 7 beträgt. Die Neutralisation ist dann erforderlich, wenn ein Fortschreiten der Hydrolyse von teilweise hydrolysierten Polymerisaten vermieden oder verzögert werden soll. Die Hydrolyse kann auch mit Hilfe von Enzymen vorgenommen werden.

Bei der Hydrolyse von N-Vinylcarbonsäureamideinheiten enthaltenden Copolymerisaten tritt gegebenenfalls eine weitere Modifizierung der Polymerisate dahingehend ein, daß die einpolymerisierten Comonomeren ebenfalls hydrolysiert werden. So entstehen beispielsweise aus einpolymerisierten Einheiten von Vinylestern Vinylalkoholeinheiten. In Abhängigkeit von den Hydrolysebedingungen können die einpolymerisierten Vinylester vollständig oder partiell verseift werden.

Bei einer partiellen Hydrolyse von Vinylacetateinheiten enthaltenden Copolymeren umfasst das hydrolysierte Copolymerisat neben unveränderten Vinylacetateinheiten Vinylalkoholeinheiten sowie N-Vinylcarbonsäureamid- und Vinylamineinheiten. Aus Einheiten monoethylenisch ungesättigter Carbonsäureanhydride, -ester und amide entstehen bei der Hydrolyse Carbonsäureeinheiten. Einpolymerisierte, monoethylenisch ungesättigte Carbonsäuren selbst werden bei der Hydrolyse nicht verändert. Auch aus einpolymerisierten, monoethylenisch ungesättigten Nitrilen können Carbonsäureamid- und Carbonsäureeinheiten gebildet werden. Der Hydrolysegrad der einpolymerisierten Comonomeren kann analytisch leicht bestimmt werden.

Die Aminfunktionen können sowohl in Form der freien Basen oder neutralisiert mit Mineralsäuren oder organischen Säuren, wie beispielsweise Ameisensäure, Essigsäure, Valeriansäure, 2-Ethylhexansäure, Laurinsäure, Adipinsäure, Benzoesäure, p-Methoxybenzoesäure, Milchsäure, Citronensäure, in Salzform vorliegen. Weiterhin besteht die Möglichkeit, unsubstituierte Amingruppen zu alkylieren bzw. zu quaternisieren. Die Alkylierung und Quaternisierung erfolgt dabei mit Hilfe gängiger Alkylierungsmittel, wie Alkylhalogeniden, z.B. Methylchlorid, Methylbromid, Methyljodid, Ethylbromid, Ethyljodid oder Benzylbromid, oder auch Dimethylsulfat oder Diethylsulfat. Die Reaktion kann dabei in wäßrigem Medium, in inerten organischen Lösungsmitteln oder auch in Mischungen von Wasser mit Lösungsmitteln, gegebenenfalls unter Phasentransferkatalyse, erfolgen. Vorzugsweise führt man die Alkylierung und Quaternisierung in wäßrigem Medium durch.

Die erfindungsgemäß verwendeten Copolymerisate haben Molekulargewichte von 1000 bis 10 Millionen, vorzugsweise 10 000 bis 5 Millionen, was K-Werten von etwa 5 bis 300 bzw. 10 bis 250 entspricht, gemessen an 1 %-igen wäßrigen Lösungen bei pH 7 und 25°C nach H. Fikentscher, Cellulose-Chemie, Band 13, 58 bis 64 und 71 bis 74 (1932).

Die zuvor beschriebenen Copolymerisate finden Anwendung als Wirkstoffe in kosmetischen Zubereitungen, beispielsweise als Conditioner für Shampoos, Haarkuren, Lotionen, Emulsionen, Spülungen, Gele, Schäume und Vor- und Nachbehandlungsmittel für Haarfärbung und Dauerwelle sowie als Haarfestiger und Haarstylingmittel mit Pflegeeigenschaften. Weiterhin können sie als Verdicker in Kosmetikformulierungen und in kosmetischen Zubereitungen für Mundpflege eingesetzt werden.

Überraschenderweise wurde gefunden, daß die erfindungsgemäßen N-Vinylformamidcopolymere verbesserte Eigenschaften in kosmetischen Formulierungen aufweisen. Am auffälligsten sind diese Eigenschaftsverbesserungen in Shampooformulierungen. Bevorzugt verwendet man die erfindungsgemäßen Copolymere in marktüblichen Shampooformulierungen mit Natrium- bzw. Ammoniumlaurylethersulfat als Basistensid und gegebenenfalls weiteren Cotensiden, z.B. Alkylpolyglycoside, Cocamidopropylbetaine, Sulfobernsteinsäureester, sek. Alkansulfonate, a-Olefinsulfonate, Eiweißfettsäurekondensate, N-Acylsarkosinate, Tauride, Methyltauride, Fettsäureisethionate, N-Acylglutamate, Ethercarbonsäurederivate, Alkylphosphatester, Alkylphosphatester, Alkylbetaine, Alkylamidopropalbetaine, Sulfobetaine, Alkylglycerylethersulfonate, Cocosamphocarboxyglycinate und Sobitanderivate. Nach Anwendung der N-Vinylformamidcopolymere in den oben genannten Shampoos wird insbesondere die Naßkämmbarkeit des Haares verbessert. Zudem wird dem Haar Glanz, Volumen und Body sowie hervorragende Festigkeit verliehen. Die Shampoos haben somit Wasch-, Konditionier- und Festigerwirkung (3 in 1-Shampoos), wobei die Polymeren schon in niedrigen Einsatzmengen zur Wirkung kommen.

Gute Konditionier- und Festigungseffekte zeigen die erfindungsgemäßen Copolymere auch in Haarkuren, Lotionen, Emulsionen, Spülungen sowie Stylingmitteln, wie Gelen und Schäumen, und in Vor- und Nachbehandlungsmitteln für Haarfärbung und Dauerwelle, wobei sie hervorragende Pflegeeigenschaften bewirken.

Weiterhin können die Copolymeren auch als Konditioniermittel und Verdicker in Hautpflegemitteln, wie Cremen, Salben, Emulsionen und Lotionen sowie für die Mundpflege in Zahnpasten, Gelen und Mundwässern eingesetzt werden.

Gegenstand der Erfindung sind somit auch kosmetische Mittel, enthaltend wenigstens ein Polymerisat gemäß obiger Definition in einem kosmetischen Träger und gegebenenfalls in Kombination mit weiteren kosmetisch aktiven Substanzen.

Die erfindungsgemäßen Polymerisate sind in den kosmetischen Mitteln gewöhnlich in einem Anteil von etwa 0,01 bis 15 Gew.-%, wie z.B. etwa 0,1 bis 10 Gew.-%, bezogen auf das Gesamtgewicht des Mittels, enthalten. Neben üblichen kosmetischen Trägern können weitere übliche Zusätze, wie z.B. oberflächenaktive Mittel, Verdickungsmittel, gelbildende Mittel, Lösungsvermittler, Feuchthaltemittel, Bindemittel, Treibmittel, Polymere, wie z.B. Silikone, Sequestriermittel, Chelatbildner, Viskositätsmodifikatoren, Trübungsmittel, Stabilisatoren, Perlglanzmittel, Farbstoffe, Duftstoffe, organische Lösungsmittel, Konservierungsmittel, pH-einstellende Mittel und gegebenenfalls weitere Konditioniermittel enthalten sein.

Die Erfindung wird nun anhand der folgenden, nicht limitierenden Beispiele näher erläutert.

Herstellungsbeispiel

Herstellung von Copolymer aus 90 Mol-% N-Vinylformamid und 10 Mol-% N-Vinyl-N'-methyl-imidazoliumchlorid (Copolymer 1).

Eine Mischung aus 180 g N-Vinylformamid, 44,4 g einer 45%igen wässrigen Lösung von N-Vinyl-N'-methylimidazoliumchlorid und 775,6 g Wasser wurde unter Stickstoff in einer Laborapparatur mit Rückflußkühler und Ankerrührer auf 70°C erhitzt. Nach Zugabe einer Lösung von 0,6 g 2,2'-Azobis(2-amidinopropan)dihydrochlorid in 10 ml Wasser wurde 8 Stunden unter Rühren die Temperatur von 70°C gehalten. Dabei wurde durch Zugabe von Ammoniak während der Polymerisation ein Absinken des pH-Wertes unter einen Wert von 7 verhindert. Nach Abkühlen der Reaktionsmischung wurde eine klare, farblose Polymerlösung mit einem Feststoffgehalt von 20,1 % erhalten. Der K-Wert des Polymers (gemessen 0,1%ig in 5%iger Kochsalzlösung) betrug 104. Die Bestimmung des K-Wertes ist beschrieben in H. Fikentscher "Systematik der Cellulosen auf Grund ihrer Viskosität in Lösung", Cellulose-Chemie 13, (1932) 58-64 und 71-74.

Anwendungsbeispiel

Das Copolymer 1 gemäß Herstellungsbeispiel wurde bezüglich seiner Haarkonditionierwirkung mit den bekannten Copolymeren 2 und 3 verglichen.

Copolymer 2 (Stand der Technik)

Handelsübliches Copolymerisat aus Acrylamid und Diallyldimethylammoniumchlorid mit einem Molekulargewicht von ca. 1 000 000 (Merquat S von Merck Calgon).

Copolymer 3 (Stand der Technik)

Handelsübliche kationische Hydroxyethylcellulose (Celquat H 100 von National Starch & Chem. Inv. Hold. Corp.).

Zur Prüfung als Konditioniermittel für Haarshampoos wurden die genannten Copolymerisate 1 bis 3 in angegebener Menge einem standardmäßigen Testshampoo mit 15,0 Gew.-% Natriumlaurylethersulfat mit 2 bis 3 Ethylenoxideinheiten (Texapon NSO von Henkel KG) zugesetzt. Anhand von definierten Haartressen wurde der Einfluß der Polymeren auf die Naßkämmbarkeit getestet. Dazu wurden die Haartressen mit dem polymerhaltigen Testshampoo gewaschen und gespült und dann die Kämmkraft gemessen. Als Blindprobe diente eine Haartresse, die mit Testshampoo ohne Zusatz behandelt wurde. Die Ergebnisse sind in Tabelle 1 aufgeführt.

Tabelle 1:

| Copolymer | Zusatzmenge [% tel.qel] | Kämmkraftabnahme [bzgl. Blindwert] |
|---|---|---|
| 1 | 0,1 | 43 |
| 2 | 0,1 | 5 |
| | 1,0 | 18 |
| 3 | 0,1 | 25 |

Die Kämmkraftabnahme errechnet sich nach folgender Formel:

$$\text{Abnahme [\%]} = \left[\frac{\text{Meßwert x 100}}{\text{Blindwert}}\right] - 100$$

Demnach sind hohe Kämmkraftabnahmen als positiv zu bewerten.

## Patentansprüche

**1.** Verwendung eines wasserlöslichen Copolymers, umfassend als charakteristische Strukturelemente

a) Vinylcarbonsäureamid-Einheiten der allgemeinen Formel I

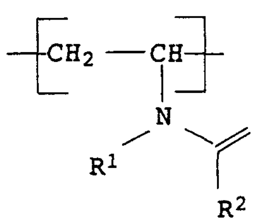

(I)

worin
$R^1$ und $R^2$ unabhängig voneinander für H, Alkyl, Cycloalkyl, Aryl oder Aralkyl stehen, und

b) heterocyclische Einheiten, ausgewählt unter

i)

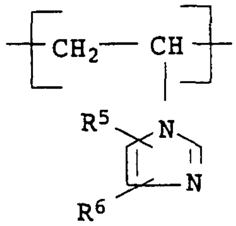

(III)

worin
$R^5$ und $R^6$ unabhängig voneinander ausgewählt sind unter H, Alkyl, Aryl, Aralkyl oder zusammen einen benzannelierten Ring bedeuten; und

ii)

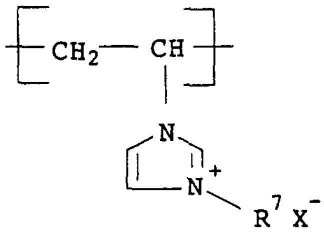

(IV)

worin
X für den Rest einer anorganischen oder organischen Säure steht, und $R^7$ für H, Alkyl oder Aralkyl steht;

als Bestandteil kosmetischer Mittel.

**2.** Verwendung nach Anspruch 1, **dadurch gekennzeichnet, daß** das Copolymer ein gewichtsmittleres Molekulargewicht im Bereich von $10^3$ bis $10^8$ g/mol besitzt.

**3.** Verwendung nach einem der vorhergehenden Ansprüche, **dadurch gekennzeichnet, daß** das Copolymer

a) 0,1 bis 99,9 Mol-% N-Vinylcarbonsäureamid-Einheiten der Formel I,
b) 0,1 bis 99,9 Mol-% heterocyclische Einheiten der Formel III und/oder IV,
c) 0 bis 99,8 Mol-% von a) und b) verschiedener monoethylenisch ungesättigter Monomereinheiten; und
d) 0 bis 5 Mol-% Monomereinheiten mit mindestens zwei ethylenisch ungesättigten Doppelbindungen

umfaßt.

**4.** Verwendung nach Anspruch 3, **dadurch gekennzeichnet, daß** die Monomer-Einheiten c) abgeleitet sind von Vinylalkohol, Vinylformiat, Vinylacetat, Vinylpropionat, Vinylbutyrat, N-Vinylpyrrolidon, N-Vinylcaprolactam, N-Vinylharnstoffen, $C_1$-$C_6$-Vinylethern, monoethylenisch ungesättigten $C_3$-$C_8$-Carbonsäuren und -Dicarbonsäuren sowie deren Estern, Amiden, Anhydriden und Nitrilen, Olefinen, Vinylaromaten, Vinylaminen, Vinylammoniumhalogeniden und Sulfonsäuregruppen enthaltenden Monomeren mit polymerisierbarer Doppelbindung.

**5.** Verwendung eines Copolymers nach einem der Ansprüche 1 bis 4 als Konditionier- oder Verdickungsmittel.

**6.** Verwendung nach einem der vorhergehenden Ansprüche, **dadurch gekennzeichnet, daß** das kosmetische Mittel ausgewählt ist unter haarkosmetischen Mitteln, Hautpflegemitteln und Mundpflegemitteln.

**7.** Verwendung nach Anspruch 6 in Haarfestigern, Haarkuren, Lotionen, Emulsionen, Spülungen, Gelen, Schäumen, Vor- und Nachbehandlungsmitteln für Haarfärbung und Dauerwelle, Haarstylingmitteln mit Pflegeeigenschaften, oder Shampoos.

**8.** Verwendung nach Anspruch 7 als Konditioniermittel für Shampoos.

9. Kosmetisches Mittel, enthaltend wenigstens ein Copolymerisat gemäß einem der Ansprüche 1 bis 4 in einem kosmetischen Träger und gegebenenfalls in Kombination mit weiteren kosmetisch aktiven Substanzen.

## Claims

1. The use of a water-soluble copolymer which comprises as characteristic structural elements

a) vinylcarboxamide units of the general formula I

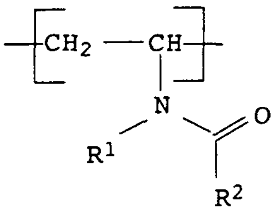

(I)

where
$R^1$ and $R^2$ independently of one another are H, alkyl, cycloalkyl, aryl or aralkyl, and

b) heterocyclic units selected from among

i)

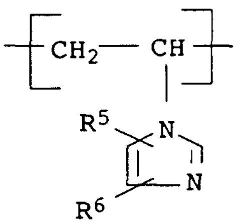

(III)

where
$R^5$ and $R^6$ independently of one another are selected from among H, alkyl, aryl, aralkyl or together are a benzo-fused ring; and

ii)

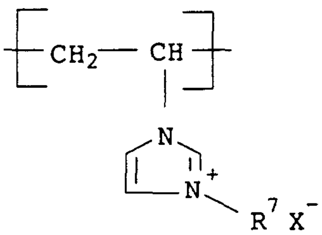

(IV)

where
X is the radical of an inorganic or organic acid and $R^7$ is H, alkyl or aralkyl;

as a constituent of cosmetic compositions.

2. The use as claimed in claim 1, wherein the copolymer has a weight-average molecular weight in the range from $10^3$ to $10^8$ g/mol.

**3.** The use as claimed in one of the preceding claims, wherein the copolymer comprises

a) from 0.1 to 99.9 mol% of N-vinylcarboxamide units of the formula I,
b) from 0.1 to 99.9 mol% of heterocyclic units of the formula III and/or IV,
c) from 0 to 99.8 mol% of monoethylenically unsaturated monomer units which are different from a) und b); and
d) from 0 to 5 mol% of monomer units having at least two ethylenically unsaturated double bonds.

**4.** The use as claimed in claim 3, wherein the monomer units c) are derived from vinyl alcohol, vinyl formate, vinyl acetate, vinyl propionate, vinyl butyrate, N-vinylpyrrolidone, N-vinyl caprolactam, N-vinyl ureas, $C_1$-$C_6$-vinyl ethers, monoethylenically unsaturated $C_3$-$C_8$-carboxylic acids and -dicarboxylic acids and also their esters, amides, anhydrides and nitriles, olefins, vinylaromatics, vinylamines, vinylammonium halides and monomers containing sulfonic acid groups having a polymerizable double bond.

**5.** The use of a copolymer as claimed in one of claims 1 to 4 as a conditioner or thickening agent.

**6.** The use as claimed in one of the preceding claims, wherein the cosmetic composition is selected from among hair cosmetic compositions, skin care compositions and oral hygiene compositions.

**7.** The use as claimed in claim 6 in setting lotions, hair treatments, lotions, emulsions, rinses, gels, foams, pre- and aftertreatment agents for hair dyeing and permanent waving, hairstyling agents having caring properties, or shampoos.

**8.** The use as claimed in claim 7 as conditioners for shampoos.

**9.** A cosmetic composition, comprising at least one copolymer as claimed in one of claims 1 to 4 in a cosmetic carrier and, if appropriate, in combination with further cosmetically active substances.

## Revendications

**1.** Utilisation d'un copolymère soluble dans l'eau contenant, en tant qu'éléments de structure caractéristiques

a) des motifs de vinylcarboxamide de formule générale I

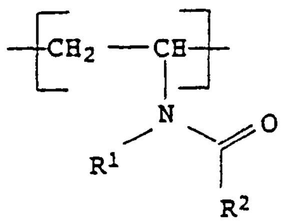

(I)

dans laquelle
$R^1$ et $R^2$ représentent chacun, indépendamment l'un de l'autre, H, un groupe alkyle, cycloalkyle, aryle ou aralkyle, et

b) des motifs hétérocycliques choisis parmi les suivants :

i)

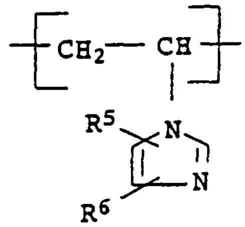

(III)

dans lesquels
$R^5$ et $R^6$ représentent chacun, indépendamment l'un de l'autre, H, un groupe alkyle, aryle, aralkyle ou représentent ensemble un cycle benzocondensé ; et

ii)

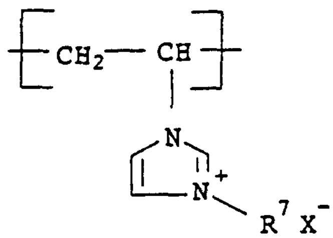

(IV)

dans lesquels
X représente le radical d'un acide minéral ou organique et $R^7$ représente H, un groupe alkyle ou aralkyle ;

en tant que constituant de produits cosmétiques.

**2.** Utilisation selon la revendication 1, **caractérisée par le fait que** le copolymère a un poids moléculaire moyen, moyenne en poids, dans l'intervalle de $10^3$ à $10^8$ g/mol.

**3.** Utilisation selon l'une des revendications qui précèdent, **caractérisé par le fait que** le copolymère contient

a) 0,1 à 99,9 mol % de motifs de N-vinylcarboxamide de formule I,
b) 0,1 à 99,9 mol % de motifs hétérocycliques de formules III et/ou IV,
c) 0 à 99,8 mol % de motifs de monomères à insaturation monoéthylénique autres que a) et b) : et
d) 0 à 5 mol % de motifs monomères à au moins deux doubles liaisons d'insaturation éthylénique.

**4.** Utilisation selon la revendication 3, **caractérisée par le fait que** les motifs de monomères c) dérivent de l'alcool vinylique, du formiate de vinyle, de l'acétate de vinyle, du propionate de vinyle, du butyrate de vinyle, de la N-vinylpyrrolidone, du N-vinylcaprolactame, de N-vinylurées, d'éthers vinyliques en C1-C6, d'acides carboxyliques et dicarboxyliques à insaturation monoéthylénique en C3-C8 ou de leurs esters, amides, anhydrides et nitriles, d'oléfines, de dérivés vinylaromatiques, de vinylamines, d'halogénures de vinylammonium et de monomères à double liaison polymérisable contenant des groupes acide sulfonique.

**5.** Utilisation d'un copolymère selon l'une des revendications 1 à 4 en tant que produit conditionnant ou épaississant.

**6.** Utilisation selon l'une des revendications qui précèdent, **caractérisée par le fait que** le produit cosmétique est choisi parmi les produits cosmétiques capillaires, les produits pour les soins de la peau et les produits pour les soins buccaux.

**7.** Utilisation selon la revendication 6 dans des fixateurs pour cheveux, des produits pour soins des cheveux, des lotions, émulsions, rinçages, gels, mousses, produits de traitement préalable et de traitement consécutif à la teinture de la chevelure et à l'ondulation permanente, produits pour l'esthétique de la chevelure, également à propriétés soignantes, ou shampooings.

**8.** Utilisation selon la revendication 7 en tant qu'agents conditionnant pour des shampooings.

**9.** Produit cosmétique contenant au moins un copolymère selon l'une des revendications 1 à 4 et un véhicule cosmétique, et le cas échéant en combinaison avec d'autres substances actives cosmétiques.